(19) Europäisches Patentamt
European Patent Office
Office européen des brevets

(11) Veröffentlichungsnummer: **0 076 445**
**B1**

(12) # EUROPÄISCHE PATENTSCHRIFT

(45) Veröffentlichungstag der Patentschrift: **29.05.85**

(51) Int. Cl.⁴: **A 01 N 43/64,** A 01 N 43/50 // C07D249/08, C07D233/58

(21) Anmeldenummer: **82108910.9**

(22) Anmeldetag: **27.09.82**

(54) **Mittel zur Regulierung des Pflanzenwachstums.**

(30) Priorität: **02.10.81 DE 3139251**

(43) Veröffentlichungstag der Anmeldung: **13.04.83 Patentblatt 83/15**

(45) Bekanntmachung des Hinweises auf die Patenterteilung: **29.05.85 Patentblatt 85/22**

(84) Benannte Vertragsstaaten: **BE CH DE FR GB IT LI NL**

(56) Entgegenhaltungen: **EP - A - 0 049 913**

(73) Patentinhaber: **BASF Aktiengesellschaft, Carl-Bosch-Strasse 38, D-6700 Ludwigshafen (DE)**

(72) Erfinder: **Meyer, Norbert, Dr., Stahlbuehlring 155a, D-6802 Ladenburg (DE)**
Erfinder: **Zeeh, Bernd, Dr., Thorwaldsenstrasse 5, D-6700 Ludwigshafen (DE)**
Erfinder: **Buschmann, Ernst, Dr., Georg-Ludwig-Krebs-Strasse 10, D-6700 Ludwigshafen (DE)**
Erfinder: **Jung, Johann, Dr., Dipl-Landwirt, Hardenburgstrasse 19, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 076 445 B1

BUNDESDRUCKEREI BERLIN

**Beschreibung**

Die vorliegende Erfindung betrifft Mittel zur Regulierung des Pflanzenwachstums, die Vinylazole oder deren Salze oder Metallkomplexe enthalten, sowie die Verwendung dieser Verbindungen zur Regulierung des Pflanzenwachstums.

Es ist bereits bekannt, stickstoffhaltige Verbindungen wie 2-Chlorethyltrimethylammoniumchlorid (CCC) (J. Biol. Chem. 235, 475 (1960)) zur Regulierung des Pflanzenwachstums zu verwenden. Mit dessen Holfe läßt sich z. B. eine Hemmung des Längenwachstums bei einigen Getreidearten und eine Hemmung des vegetativen Wachstums bei einigen anderen Kulturpflanzen erreichen. Die Wirkung dieses Stoffes ist jedoch, insbesondere bei niedrigen Aufwandmengen, nicht immer ausreichend und genügt den Anforderungen der Praxis nicht immer.

Weiterhin ist bekannt, 1-(4'-Bromphenyl)-1-allyloxy-2-(1'',2'',4''-triazolyl (1'')) ethan (A) zur Regulierung des Pflanzenwachstums von Raps, Weizen, Hafer, Roggen und Gerste zu verwenden (DE OS 2 650 831). Dessen Wirkung ist jedoch, vor allem bei niedrigen Aufwandmengen nicht immer zufriedenstellend.

Es wurde gefunden, daß Verbindungen der Formel I

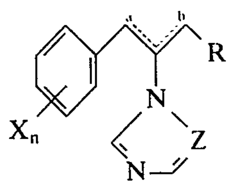

(I)

in der

R Wasserstoff, Alkyl mit 1 bis 7 Kohlenstoffatomen oder gegebenenfalls durch Halogen substituiertes Phenyl,
X Wasserstoff, Halogen, Alkyl mit 1 bis 4 Kohlenstoffatomen oder Phenyl,
Z N oder CH und
n eine ganze Zahl von 1 bis 5 bedeuten und die Doppelbindung entweder die Position a oder b einnimmt, sowie deren pflanzenverträgliche Säureadditionssalze und Metallkomplexe ausgezeichnet zur Regulierung des Pflanzenwachstums geeignet sind und eine sehr gute Pflanzenverträglichkeit zeigen.

Die Verbindungen der Formel I können als E/Z-Isomere vorliegen. Unter der Bezeichnung Vinylazole sind dementsprechend sowohl die reinen Isomeren als auch ihre Gemische zu verstehen.

In der Formel I bedeutet R Wasserstoff, einen unverzweigten oder verzweigten Alkylrest mit 1 bis 7 Kohlenstoffatomen, wie Methyl, Ethyl, Isopropyl, n-Propyl, tert.-Butyl, n-Butyl, Isobutyl, n-Pentyl, n-Hexyl, n-Heptyl, oder einen Phenylrest, der durch Halogen, wie Chlor oder Brom, einfach oder mehrfach substituiert sein kann, wie 4-Chlorphenyl, 2,4-Dichlorphenyl, 4-Bromphenyl.

X in Formel I bedeutet außer Wasserstoff oder Phenyl Halogen, wie Chlor, Brom, Iod oder einen unverzweigten oder verzweigten Alkylrest mit 1 bis 4 Kohlenstoffatomen, wie Methyl, Ethyl, n-Propyl, sec.-Butyl, Isobutyl, tert.-Butyl.

Bevorzugt sind Verbindungen der Formel I, bei denen R einen Alkylrest bedeutet und die Doppelbindung die Position a oder b einnimmt. $X_n$ bedeutet in diesen bevorzugten Verbindungen beispielsweise 2-Chlor, 4-Brom, 2,4-Dichlor, 4-Methyl, 4-Phenyl.

Die Vinylazole der Formel I erhält man, wenn man aus Verbindungen der Formeln

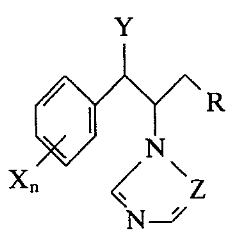

(II)

oder

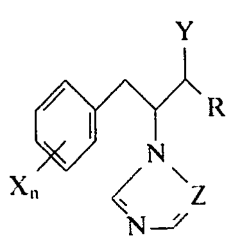

(III)

in denen R, X, Z und n die oben angegebenen Bedeutungen haben und Y eine Abgangsgruppe darstellt, HY eliminiert.

Als Abgangsgruppe für Y kommen Hydroxy, Tosylat, Mesylat, Halogenid, Acetat, Trifluoracetat, Xanthogenat in Betracht.

Bei dieser Herstellungsweise können Gemische aus Verbindungen entstehen, die hinsichtlich R, X, Z und n identisch sind, die sich aber durch die Position der Doppelbindung unterscheiden. Diese Gemische werden von Formel I umfaßt; in der Beschreibung sind sie durch die Angabe a/b zur Position der Doppelbindung charakterisiert.

Ist Y eine Hydroxygruppe, so erhält man die Vinylazole der Formel I durch Eliminierung von Wasser in Gegenwart eines sauren Dehydratisierungskatalysators aus den entsprechenden $\alpha$-Azolylalkoholen der Formeln II bzw. III. Geeignete Katalysatoren sind Protonsäuren, Lewis-Säuren, Säureanhydride oder Säurechloride, beispielsweise Schwefel-, Phosphor- oder Ameisensäure, p-Toluolsulfonsäure, Bortrifluorid, Diphosphorpentoxid, Acetanhydrid, Phosphoroxichlorid, Thionylchlorid.

Die Mengen an saurem Katalysator beträgt 10 bis 200 Gew.-% des $\alpha$-Azolylalkohols.

Die Wasserabspaltung wird bei einer Temperatur im Bereich zwischen 70 und 180° C, vorzugsweise zwischen 80 und 120° C, durchgeführt. Sie kann in lösungsmittelfreiem Medium durchgeführt werden. Vorzugsweise arbeitet man jedoch in Gegenwart eines Lösungsmittels.

Hierfür geeignete Lösungsmittel sind Toluol, Xylol, Pyridin, Eisessig, Acetonitril.

Die Hydroxyfunktion kann auch in eine leichter abspaltbare Gruppe, z. B. in Tosylat, Halogenid oder Acetat, überführt werden. Diese Abgangsgruppe läßt sich dann in Gegenwart eines basischen Katalysators, beispielsweise eine Alkoholats, eines tertiären Amins, eines Alkalicarbonats, wie Natriummethylat, Natriumethylat, Kalium-tert.-butylat, Pyridin, Kaliumcarbonat, oder in Gegenwart von Natriumsulfid bei einer Temperatur zwischen 20 und 100° C abspalten.

Man verwendet dabei 1 bis 2,5 Mole an basischem Katalysator pro Mol Verbindung der Formel II oder III. Die Eliminierungsreaktion wird vorzugsweise in Anwesenheit eines Lösungsmittels durchgeführt. Geeignete Lösungsmittel sind aliphatische Alkohole, wie Ethanol, n-Butanol, tert.-Butanol, Octanol, chlorierte Kohlenwasserstoffe, wie Chloroform, Dichlormethan, N,N-Dialkylsäureamide, wie Dimethylformamid, sowie Dimethylsulfoxid, Pyridin, Toluol, Tetrahydrofuran, Wasser. Auch Gemische dieser Lösungsmittel können verwendet werden.

Ebenso kann die Hydroxyfunktion auch verestert, d. h. in eine Acetat- oder Xanthogenatgruppe überführt werden, die dann thermisch nach bekannten Methoden eliminiert werden kann (Houben-Weyl, Methoden der organischen Chemie, Bd. 5/1b, S. 109ff, Georg Thieme-Verlag, Stuttgart, 1972).

Die Vinylazole der Formel I können in an sich üblicher Weise in für Pflanzen verträgliche Salze oder Metallkomplexe überführt werden. Zur Salzbildung eignen sich Mineralsäuren, wie Salzsäure, Bromwasserstoffsäure, Schwefelsäure, Salpetersäure, Phosphorsäure, und organische Säuren, wie Essigsäure und Dodecylbenzolsulfonsäure. Die wachstumsregulierende Wirksamkeit der Salze der Vinylazole beruht auf dem Kation, so daß das Anion beliebig aus der Vielzahl der pflanzenphysiologisch verträglichen Anionen ausgewählt werden kann.

Metallkomplexe können durch Anlagerung der Vinylazole an die Kationen von Metallsalzen gebildet werden. Hierfür eignen sich insbesondere Kupfer-, Zink-, Eisen-, Mangan- und Nickelsalze, wie Kupfer(II)-chlorid, Kupfer(II)-sulfat, Kupfer(II)-nitrat, Zink(II)-chlorid, Eisen(III)-chlorid, Mangan(II)-chlorid, Nickel(II)-bromid.

Die als Ausgangsstoffe verwendeten Verbindungen der Formeln II und III, wobei Y Hydroxy bedeutet, lassen sich nach bekannten Verfahren aus $\alpha$-Azolylketonen herstellen (DE-OS 2 734 426).

Die folgenden Beispiele erläutern die Herstellung der Verbindungen.

## Beispiel 1

### 1-(2,4-Dichlorphenyl)-4,4-dimethyl-2-[1-(1,2,4-triazolyl)]-1-penten

49,2 g 1-(2,4-Dichlorphenyl)-4,4-dimethyl-2-[1-(1,2,4-triazolyl)]-3-pentanol (als Diastereomerengemisch) werden in 500 ml absolutem Tetrahydrofuran gelöst und unter Rühren bei Raumtemperatur mit 6,0 g 80%iger Natriumhydriddispersion versetzt. Nach achtstündigem Rühren bei 40 bis 50° C wird auf Raumtemperatur abgekühlt. Dann tropft man eine Lösung von 28,2 g Toluol-4-sulfonylchlorid in 60 ml absolutem Tetrahydrofuran zu, rührt weitere 12 Stunden bei Raumtemperatur, hydrolysiert mit Wasser, extrahiert mehrfach mit 500 ml Methylenchlorid, trocknet die organische Phase anschließend über Natriumsulfat und engt ein. Durch fraktionierte Kristallisation aus Essigester erhält man 42,5 g der diastereomeren Tosylate, die in 500 ml Dimethylsulfoxid gelöst und mit 54 g gemahlenem Natriumsulfid versetzt werden. Nach einstündigem Rühren bei Raumtemperatur gibt man 1 l Wasser zu, extrahiert zweimal mit je 1 l Diethylether, trocknet die organische Phase über Natriumsulfat und engt ein. Der ölige Rückstand wird in wenig Ethanol aufgenommen und gekühlt; dabei kristallisieren 4,5 g 1-(2,4-Dichlorphenyl)-4,4-dimethyl-2-[1-(1,2,4-triazolyl)]-1-penten vom Fp. 133° C aus (Isomer A). Durch Einengen der Mutterlauge erhält man 14,0 g 1-(2,4-Dichlorphenyl)-4,4-dimethyl-2-[1-(1,2,4-triazolyl)]-1-penten vom Sdp. 132 bis 138° C/0,05 mbar als Öl. (Isomer B)

Beispiel 2

1-(2,4-Dichlorphenyl)-2-[1-(1,2,4-triazolyl)]-2-penten

Zu einer Lösung von 107 g 1-(2,4-Dichlorphenyl)-2-[1-(1,2,4-triazolyl)]-1-pentanol in 500 ml Chloroform tropft man bei Raumtemperatur 42,9 g Thionylchlorid, läßt 12 Stunden nachrühren und erhitzt anschließend 4 Stunden unter Rückfluß. Nach aufeinanderfolgendem Waschen mit Wasser, Natriumhydrogencarbonat-Lösung und nochmals mit Wasser wird die organische Phase über Natriumsulfat getrocknet und eingeengt. Das Rohprodukt löst man in Diethylether und fällt mit Salpetersäure 34 g des Nitrats von 1-(2,4-Dichlorphenyl)-2-[1-(1,2,4-triazolyl)]-1-chlorpenten, Fp. 127° C, die bei Raumtemperatur in 400 ml tert.-Butanol mit 30,2 g Kalium-tert.-butylat 7 Stunden gerührt werden. Dann engt man ein, nimmt in Chloroform auf, wäscht mit Wasser, trocknet die organische Phase über Natriumsulfat und erhält nach Abziehen des Lösungsmittels durch Destillation 10 g 1-(2,4-Dichlorphenyl)-2-[1-(1,2,4-triazolyl)]-2-penten. Sdp. 140 bis 144° C/0,1 mbar.

Analog lassen sich beispielsweise folgende Vinylazole der Formel I herstellen:

| Nr. | R | $X_n$ | Z | Position der Doppelbindung | Fp/Kp [° C] |
|---|---|---|---|---|---|
| 3 | tert.-$C_4H_9$ | 4-Cl | N | a | 78—90 |
| 4 | tert.-$C_4H_9$ | 4-Br | N | a | 133—140 |
| 5 | tert.-$C_4H_9$ | 4-$CH_3$ | N | a/b | 52—59 |
| 6 | tert.-$C_4H_9$ | H | N | a/b | 110—112/0,2 mbar |
| 7 | tert.-$C_4H_9$ | 4-$C_6H_5$ | N | a | 92—98 |
| 8 | n-$C_3H_7$ | 2-Cl, 4-Cl | N | a/b | 152—156/0,13 mbar |
| 9 | $C_2H_5$ | 2-Cl, 4-Cl | CH | a/b | 170—190/0,4 mbar |
| 10 | tert.-$C_4H_9$ | 2-Cl, 4-Cl | CH | a | 135/0,01 mbar |
| 11 | i-$C_3H_7$ | 2-Cl, 4-Cl | N | a/b | 148—152/0,08 mbar |
| 12 | tert.-$C_4H_9$ | 2-I | N | a/b | 69/78 |
| 13 | tert.-$C_4H_9$ | 2-Cl, 3-Cl, 4-Cl | N | a/b | Harz |
| 14 | tert.-$C_4H_9$ | 2-Cl, 4-Cl | N | a/b | 142—145/0,2 mbar |
| 15 | H | 2-Cl, 4-Cl | CH | a/b | 152—158 (als Nitrat) |
| 16 | n-$C_5H_{11}$ | 2-Cl, 4-Cl | N | a/b | 160—170/0,02 mbar |
| 17 | n-$C_6H_{13}$ | 2-Cl, 4-Cl | N | a/b | 153—167/0,01 mbar |
| 18 | 2,4-Dichlorphenyl | 2-Cl, 4-Cl | N | a | Harz |
| 19 | 2,4-Dichlorphenyl | 2-Cl, 4-Cl | CH | a | Harz |
| 20 | 4-Chlorphenyl | 2-Cl, 4-Cl | N | a/b | Harz |
| 21 | tert.-$C_4H_9$ | 2-I | CH | a/b | 172/0,01 mbar |
| 22 | n-$C_5H_{11}$ | 2-Cl, 4-Cl | CH | a/b | 162/185/0,15 mbar |

Die Verbindungen der Formel I greifen in den Stoffwechsel der Pflanzen ein und können deshalb als Wachstumsregulatoren eingesetzt werden.

Für die Wirkungsweise von Pflanzenwachstumsregulatoren gilt nach den bisherigen Erfahrungen, daß ein Wirkstoff eine oder auch mehrere verschiedenartige Wirkungen auf Pflanzen ausüben kann.

Die Wirkungsvielfalt der Pflanzenwachstumsregulatoren hängt ab vor allem

a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation),
d) von den geoklimatischen Faktoren, z. B. Sonnenscheindauer, Durchschnittstemperatur, Niederschlagsmenge,
e) von der Bodenbeschaffenheit (einschließlich Düngung),
f) von der Formulierung bzw. Anwendungsform des Wirkstoffs und schließlich
g) von der angewendeten Konzentration der aktiven Substanz.

In jedem Falle sollen Wachstumsregulatoren die Kulturpflanze in gewünschter Weise positiv beeinflussen.

Aus der Reihe der verschiedenartigen Anwendungsmöglichkeiten der erfindungsgemäßen Pflanzenwachstumsregulatoren im Pflanzenanbau, in der Landwirtschaft und im Gartenbau werden einige nachstehend erwähnt.

A. Mit den Verbindungen der Formel I läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z. B. die Verringerung des Grasbewuchses an Straßenrändern, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachten Halmverkürzung und Halmverstärkung verringern oder beseitigen die Gefahr des »Lagerns« (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z. B. bei Tabakpflanzen die Ausbildung von Seitentrieben (Geiztrieben) zugunsten des Blattwuchses gehemmt werden soll.

Ein weiterer Mechanismus der Ertragssteigerung mit Wachstumshemmern beruht darauf, daß die Nährstoffe in stärkerem Maße der Blüten- und Fruchtbildung zugutekommen, während das vegetative Wachstum eingeschränkt wird. Ferner kann so wegen der relativ geringen Blatt- bzw. Pflanzenmasse dem Befall mit verschiedenen insbesondere pilzlichen Krankheiten vorgebeugt werden.

Die Hemmung des vegetativen Wachstums ermöglicht außerdem bei vielen Kulturpflanzen eine dichtere Bepflanzung, so daß ein Mehrertrag, bezogen auf die Bodenfläche erzielt werden kann.

Die erfindungsgemäßen Verbindungen eignen sich besonders zur Hemmung des vegetativen Wachstums bei Kulturpflanzen wie Soja, Sonnenblumen, Erdnüssen, Raps, Zierpflanzen, Baumwolle, Reis und Gräsern.

B. Mit den Wirkstoffen der Formel I lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu Steigern oder Gummibäume zum vermehrten Latexfluß zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven

oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt und Sproßteil der Pflanze ist auch für ein gut kontrollierbares Entblättern der Bäume wesentlich.

Die Wirkung der Verbindungen der Formel I ist besser als bei bekannten Wachstumsregulatoren. Die Wirkung zeigt sich sowohl bei Monokotylen, z. B. Getreide wie Weizen, Gerste, Roggen, Hafer und Reis oder Mais oder Gräsern als auch insbesondere bei Dikotylen (z. B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Die Wirkstoffe der Formel I können den Kulturpflanzen sowohl vom Samen her (als Saatgutbeizmittel) als auch über den Boden, d. h. durch die Wurzel sowie — besonders bevorzugt — durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,001 bis 12 kg/ha bevorzugt 0,01 bis 3 kg/ha als ausreichend zu betrachten.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin), Dimethylformamid und Wasser; feste Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung oder in einer Lösung von Wasser unter Zusatz von mit Wasser mischbaren organischen Lösungsmitteln wie Methanol oder anderen niederen Alkoholen, Aceton, Dimethylformamid oder N-Methylpyrrolidin. Die Formulierungen enthalten im allgemeinen zwischen 0,1 und 95 Gewichtsprozent Wirkstoff, vorzugsweise zwischen 0,5 und 90.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Die erfindungsgemäßen Mittel können in diesen Aufwendungsformen auch zusammen mit anderen Wirkstoffen vorliegen, wie z. B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrößerung des Wirkungsspektrums. Bei einer Anzahl solcher Wachstumsregulatormischungen treten auch synergistische Effekte auf, d. h. die Wirksamkeit des Kombinationsproduktes ist größer als die addierten Wirksamkeiten der Einzelkomponenten.

In den nachfolgenden Versuchen wird die Wirkung der erfindungsgemäß verwendbaren Stoffe als Pflanzenwachstumsregulatoren dargestellt, ohne die Möglichkeit weiterer Anwendungen als Wachstumsregulatoren auszuschließen.

## Beispiel A

### Gewächshausversuch

Zur Bestimmung der wachstumsregulierenden Eigenschaften der Prüfsubstanzen wurden Testpflanzen auf ausreichend mit Nährstoffen versorgtem Kultursubstrat in Kunststoffgefäßen von ca. 12,5 cm Durchmesser angezogen.

Im Vorauflaufverfahren wurden die Testsubstanzen in wäßriger Aufbereitung am Tage der Einsaat auf das Saatgut gegossen.

Im Vorauflaufverfahren zeigten an Sommergerste beispielsweise die Substanzen der Beispiele 4 und 11 und an Sommerraps die Substanzen der Beispiele 2 bis 4, 9 und 11 eine bessere Wirkung als die Vergleichssubstanzen. Dasselbe gilt im Nachauflaufverfahren an Sommerweizen für die Substanz des Beispiels 4 an Sommergerste für die Substanzen der Beispiele 2 bis 4, 8, 9, an Sonnenblumen für die Substanzen der Beispiele 2 bis 4, 8, 9, 11, 16, 17, 22, an Sommerraps für die Substanzen der Beispiele 2 bis 4, 8, 9, 11, 12, 16, 17 und an Soja für die Substanzen der Beispiele 2 bis 4, 8, 9 und 11.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

**Patentansprüche**

1. Mittel zur Regulierung des Pflanzenwachstums enthaltend eine Verbindung der Formel I

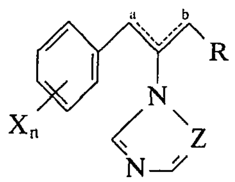

(I)

in der

R Wasserstoff, Alkyl mit 1 bis 7 Kohlenstoffatomen oder gegebenenfalls durch Halogen substituiertes Phenyl,
X Wasserstoff, Halogen, Alkyl mit 1 bis 4 Kohlenstoffatomen oder Phenyl,
Z N oder CH und
n eine ganze Zahl von 1 bis 5 bedeuten und die Doppelbindung entweder die Position a oder b einnimmt, sowie deren pflanzenverträgliche Säureadditionssalze und Metallkomplexe.

2. Mittel nach Anspruch 1, enthaltend Vinyltriazole der Formel I, dadurch gekennzeichnet, daß R Alkyl mit 1 bis 7 Kohlenstoffatomen bedeutet und die Doppelbindung die Position a einnimmt.
3. Mittel nach Anspruch 1, enthaltend Vinyltriazole der Formel I, dadurch gekennzeichnet, daß R Alkyl mit 1 bis 7 Kohlenstoffatomen bedeutet und die Doppelbindung die Position b einnimmt.
4. Mittel zur Regulierung des Pflanzenwachstums, enthaltend eine oder mehrere Verbindungen der Formel I gemäß Anspruch 1 und einem flüssigen oder festen Trägerstoff.
5. Verwendung von Verbindungen der Formel I, gemäß Anspruch 1 zur Regulierung des Pflanzenwachstums.
6. Verfahren zur Regulierung des Pflanzenwachstums, dadurch gekennzeichnet, daß man eine oder mehrere Verbindungen der Formel I gemäß Anspruch 1 auf Pflanzen oder deren Lebensraum einwirken läßt.

**Claims**

1. An agent for regulating plant growth, containing a compound of the formula I

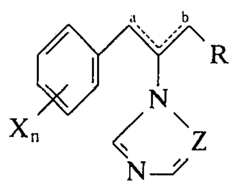

(I)

where R is hydrogen, alkyl of 1 to 7 carbon atoms, phenyl or halophenyl, X is hydrogen, halogen, alkyl of 1 to 4 carbon atoms or phenyl, Z is N or CH, and n is an integer from 1 to 5, and the double bond is in either position a or position b, and plant-tolerated acid addition salts or metal complexes thereof.
2. An agent as claimed in claim 1, containing vinyltriazoles of the formula I, wherein R is alkyl of from 1 to 7 carbon atoms and the double bond is in position a.
3. An agent as claimed in claim 1, containing vinyltriazoles of the formula I, wherein R is alkyl of from 1 to 7 carbon atoms and the double bond is in position b.
4. An agent for regulating plant growth, containing one or more compounds of the formula I as defined in claim 1 and a solid or liquid carrier.
5. The use of compounds of the formula I as defined in claim 1 for regulating plant growth.
6. A process for regulating plant growth, wherein one or more compounds of the formula I as defined in claim 1 are allowed to act on plants or their habitat.

**Revendications**

1. Agents de régularisation de la croissance des plantes, contenant un composé de formule I

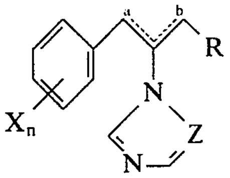

(I)

dans laquelle:

R représente hydrogène, alkyle à 1 à 7 atomes de carbone ou éventuellement phényle substitué par halogène,
X représente hydrogène, halogène, alkyle à 1 à 4 atomes de carbone ou phényle,
Z représente N ou C et
n un nombre entier de 1 à 5 et la double liaison occupe la position a ou b,

ainsi que leurs sels d'addition d'acide et complexes métalliques acceptables pour les plantes.

2. Agents selon la revendication 1, contenant des vinyltriazoles de formule I, caractérisés par le fait que R représente alkyle à 1 à 7 atomes de carbone et la double liaison occupe la position a.

3. Agents selon la revendication 1, contenant des vinyltriazoles de formule I, caractérisés par le fait que R représente alkyle à 1 à 7 atomes de carbone et la double liaison occupe la position b.

4. Agents de régularisation de la croissance des plantes contenant un ou plusieurs composés de formule I, selon la revendication 1 et un support liquide ou solide.

5. Utilisation de composés de formule I, selon la revendication 1 pour la régularisation de la croissance des plantes.

6. Procédé de régularisation de la croissance des plantes, caractérisé par le fait que l'on fait agir un ou plusieurs composés de formule I, selon la revendication 1, sur des plantes ou leur biotope.